# EUROPEAN PATENT SPECIFICATION

(11) **EP 1 547 576 B1**
(45) Date of publication and mention of the grant of the patent: **05.08.2009**
(21) Application number: 04028358.2
(22) Date of filing: 30.11.2004
(51) Int. Cl.: A61K 8/36, A61Q 15/00

(54) **Antiperspirant and deodorant stick composition**
Schweisshemmende und desodorierende Zusammensetzung in der Form eines Stiftes
Composition antisudorale et déodorante en bâtonnet

(30) Priority: 24.12.2003 US 744046
(43) Date of publication of application: 29.06.2005
(73) Proprietor: KAO CORPORATION, Tokyo 103-8210 (JP)
(72) Inventor: Takeuchi, Katsuhiko, Tokyo 131-8501 (JP); Uzu, Atsushi, Tokyo 131-8501 (JP)
(74) Representative: HOFFMANN EITLE

(56) References cited:
- EP-A- 0 550 960
- WO-A-02/102337
- WO-A-03/088942
- FR-A- 2 770 845
- US-A- 4 724 139

## Description

### Field of the Invention

The present invention relates to a topical composition containing an antiperspirant active. Particularly, the present invention relates to an antiperspirant and/or deodorant composition in a solid form.

### Background of the Invention

Many solid form antiperspirant and deodorant compositions are available in the chemical and cosmetic literature. These products typically contain an astringent material, e.g., zirconium or aluminum salts or combinations thereof, solubilized or dispersed in a suitable liquid carrier, and the solution or dispersion are contained within a solid matrix that gives the product a solid or "stick" form. These solid antiperspirant and deodorant stick compositions are ideally designed to provide effective control of perspiration and odor while having good consumer acceptance due to the ease of application to the skin, good cosmetic aesthetics and an acceptable degree of effectiveness. In this context, good consumer acceptance means that the product glides on smoothly during application, is non-irritating, and results in little or no visible residue after application to the skin.

In order to create a solid form, many types of structurant systems may be formed to provide acceptable physical properties. One type is composed of a soap/alcohol gel structurant, but such form may have stability problems such as unstable gel structures, which render such sticks less aesthetically pleasing to consumers. In another type, waxy materials are used as a structurant. The most commonly used waxy material in sticks of this type includes long-chain fatty alcohols. Such alcohols are sometimes used in combination with volatile silicones. Although these fatty alcohol/volatile silicone-based solid sticks avoid some of the soap gel stability problems, they may vary unacceptably in structural hardness and strength. Using a large amount of waxy structurant may not be necessarily effective since it may result in an overly hard structure. Therefore, formulations are sought which provide hard fatty alcohol/volatile silicone-based sticks without causing those sticks to become too brittle or hard and cosmetically unacceptable. The hardness of the solid forms is closely related to qualities such as the strength against breakage, storage stability (especially at higher temperatures), and product aesthetics such as smooth application/gliding on the skin, and drier feel upon use. Such unacceptable solid form may also tend to leave an unsightly white chalky residue on the skin after application. This residue is not only aesthetically displeasing to the user but can also soil clothing. Thus, the control of stick hardness is important to improve the overall product quality.

Investigations have been conducted to improve the qualities, properties and aesthetics of solid antiperspirant/deodorant sticks. For example, in U.S. Pat. No. 4,725,432 (May), long chain fatty alcohols having a chain length of C₂₀ or longer was used in order to improve the stick hardness of solid sticks.

EP 0 550 960 A discloses an antiperspirant composition for use as a cosmetic stick which comprises glyceryl monooleate, an antiperspirant active and structurants.

US 4,724,139 describes an antiperspirant stick composition comprising an antiperspirant active, emollients such as oleic acid and water-insoluble waxes. The emollient is present in an amount of at least 1.0 %.

WO 03/088942 A is related to a cosmetic composition for topical application to human skin comprising hydroxyphenyl alkyl derivatives of C₁₀ to C₁₆ unsaturated carboxylic acids.

WO 02/102337 A relates to antiperspirant or deodorant compositions for topical application to human skin comprising an antiperspirant active, a carrier and a fatty acid activating peroxisome proliferators-activated receptors, for instance, olefinically unsaturated acids having 16 or 18 carbon atoms. The purpose of these compositions is the amelioration or control of skin irritancy.

There is a continuing need and desire to improve the formulation of anti-perspirant and deodorant stick compositions such that it has satisfactory strength and hardness while exhibiting aesthetically pleasing attributes.

### Summary of the Invention

The present invention relates to a topical stick composition containing an antiperspirant active, a structurant, and from 0.2% to 0.7% of an unsaturated fatty acid. Preferably, the amount of antiperspirant active is from 0.5% to 60% by weight and the amount of structurant is from 3% to 35% by weight, based on the topical composition.

### Detailed Description of the Invention

The present invention relates to topical compositions containing an antiperspirant active, a structurant selected from stearyl alcohol, cetyl alcohol, arachidyl alcohol, behenyl alcohol and hydrogenated castor oil, and mixtures thereof and a select amount of an unsaturated fatty acid. The present invention relates to such compositions in a solid form, which have satisfactory strength and hardness while exhibiting aesthetically pleasing attributes. It has been surprisingly found that with the addition of a select amount of an unsaturated fatty acid, the hardness and strength of such topical stick composition is improved while achieving satisfactory consumer acceptance. Satisfactory consumer acceptance includes ease of application to the skin, good cosmetic aesthetics, non-irritating, acceptable degree of effectiveness, and results in little or no visible residue after application to the skin. The resultant stick also has a pleasant odor to the consumer when used.

Although the use of higher amounts of unsaturated fatty acids in antiperspirant and deodorant compositions are well established, for example as emollients, it has been discovered that the select amount of from 0.2% to 0.7% of an unsaturated fatty acid properly adjusts the physical properties of the stick relevant to hardness and strength. Especially for preferred solid forms containing waxy materials as a structurant, the resultant stick is sufficiently hard without the need to increase the amount of waxy materials in the formulation. The hardness is preferably selected for each topical stick formulation to help provide the desired application rheology, thus resulting in the desired use feeling and low-residue application as applied to the skin. In addition, such highly aesthetic properties are achieved in a lower cost formulation. Although a preferred use of these stick compositions is for antiperspirant sticks, they may also be used as deodorant sticks as well as other cosmetic vehicles. Furthermore, although some unsaturated fatty acids may have an unpleasant odor and may be undesirable to formulate into antiperspirant and/or deodorant compositions, at the select amount, such odor upon application is not compromised.

All percentages are by weight % of the total composition, unless indicated otherwise in the description.

The term "anhydrous" as used herein refers to compositions or materials, including the preferred anhydrous embodiments of the present invention, that contain less than about 10%, more preferably less than about 8%, even more preferably less than about 5%, and even more preferably less than about 3% by weight of free or added water, other than the water of hydration typically associated with any particulate solids prior to formulation.

All values, amounts and measurements described herein are obtained under ambient conditions unless otherwise specified. The term "ambient conditions" as used herein refers to conditions under about one (1) atmosphere of pressure, at about 50% relative humidity, and at about 25°C, unless otherwise specified.

The term "volatile" as used herein refers to those materials that have a measurable vapor pressure at 25°C. Such vapor pressures will typically range from about 0.01 mm Hg to about 6 mm Hg, more typically from about 0.02 mm Hg to about 1.5 mm Hg, and have an average boiling point at one (1) atmosphere pressure (atm) of less than about 250°C. Conversely, the term "non-volatile" refers to those materials that are not "volatile" as defined therein.

As used herein, the term "solid" is intended to encompass compositions which typically have hardness values above about 100 grams, generally above about 200 grams, using conventional devices. In a typical method known in the art, these hardness values can be measured at 25°C, 40% relative humidity using a FUDOH RHEO METER NRM-2010J-CW, available from RHEOTECH Corp., Bunkyo-ku, Tokyo, JAPAN. The standard penetration plunger is smooth, made of stainless steel and has a diameter of 3 mm. It is available from RHEOTECH Corp., as adapter number 6. The hardness value represents the peak force required to move the standard plunger from the surface through the core of the stick composition, for a distance of 10 mm at a rate of 2 cm/minute.

### Unsaturated Fatty Acids

The topical stick composition of the present invention contains a select amount of unsaturated fatty acid so as to provide a stick having appropriate hardness and good physical properties. The topical stick composition contains from 0.2% to 07% of an unsaturated fatty acid, preferably from 0.1 % to 0.7%, more preferably from 0.2% to 0.6%, and more preferably from 0.2% to 0.5%, by weight of the topical stick composition. It has been discovered that with the addition of such optimum range of unsaturated fatty acids, the topical stick composition provides aesthetically pleasing properties, including having satisfactory glide, substantially no greasy feel, and a low degree of fabric staining due to white, chalky residue.

Preferred unsaturated fatty acids include for example, carboxylic acids having from about 9 to about 30 carbon atoms. Exemplary carboxylic acids include, oleic acid, linoleic acid, linolenic acid, palmitoleic acid, and mixtures thereof. Such fatty acids or fatty acid mixtures may be derived from natural fats and oils such as tung oil, safflower oil, coconut oil, corn oil, cottonseed oil, fish oil, whale oil, sunflower oil, sesame seed oil, linseed oil, castor oil, rice germ oil, and tallow. Preferred carboxylic acids include oleic acid, linoleic acid, and linolenic acid, including their mixtures, wherein oleic acid is especially preferred.

### Antiperspirant active

The topical stick compositions of the present invention contain an antiperspirant active suitable for application to human skin. The concentration of the active should be sufficient to provide the desired control of perspiration wetness or odor from the formulation selected.

The compositions of the present invention preferably contain an antiperspirant active at concentrations ranging from 0.5% to 60%, more preferably from 5% to 40%, even more preferably from 10% to 30%, by weight of the composition. These weight percentages are calculated on an anhydrous metal salt basis exclusive of water and any complexing agents such as glycine, glycine salts, or other complexing agents. The antiperspirant active is preferably a solid. The antiperspirant active as formulated in the composition is preferably in the form of dispersed particulate solids having a preferred average particle size or diameter of less than about 100 micrometers, more preferably less than about 20 micrometers, and even more preferably less than about 10 micrometers. A preferred average particle size or diameter range is from about 0.1 micron to 100 micrometers, preferably from 0.1 micrometer to 20 micrometers, and even more preferably from about 0.1 micrometer to 10 micrometers. They may be impalpable or microspherical in form and, preferably, have a high bulk density (for example, greater than about 0.7 g/cm³). Any known antiperspirant active may be used in the present invention.

Preferred antiperspirant actives include astringent metallic salts, particularly inorganic and organic salts of aluminum, zirconium and zinc, as well as mixtures thereof. Even more preferred are aluminum-containing and/or zirconium-containing salts or materials, such as aluminum halides, aluminum chlorohydrate, aluminum hydroxyhalides, zirconyl oxyhalides, zirconyl hydroxyhalides, and mixtures thereof.

Preferred aluminum salts of the present invention include:

Al₂(OH)ₐCl_{b}^{.}xH₂O

wherein a is from about 2 to 5; the sum of a and b is about 6; x is from about 1 to 6; and wherein a, b, and x may have non-integer values. Particularly preferred are the aluminum chlorohydroxides referred to as "5/6 basic chlorohydroxide", wherein a=4. Processes for preparing aluminum salts are disclosed for example in U.S. Pat. No. 3,887,692 (Gilman), and U.S. Pat. No. 3,904,741 (Jones et al.). Mixtures of aluminum salts are described in GB 2,048,229.

Preferred zirconium salts include:

ZrO(OH)₂₋ₐClₐ·xH₂O

wherein a is from about 1.5 to 1.897; x is from about 1 to 7; and wherein a and x may both have non-integer values. Also preferred zirconium salts are those complexes which additionally contain aluminum and glycine, commonly known as ZAG complexes such as those described in U.S. Pat. No. 3,792,068 (Luedders et al.) and U.S. Pat No. 4,120,948 (Shelton et al.). ZAG complexes are chemically analyzable for the presence of aluminum, zirconium and chlorine.

Antiperspirant actives suitable for use in the compositions include aluminum chlorohydrate, aluminum dichlorohydrate, aluminum sesquichlorohydrate, aluminum chlorohydrex propylene glycol complex, aluminum dichlorohydrex propylene glycol complex, aluminum sesquichlorohydrex propylene glycol complex, aluminum chlorohydrex polyethylene glycol complex, aluminum dichlorohydrex polyethylene glycol complex, aluminum sesquichlorohydrex polyethylene glycol complex, aluminum zirconium trichlorohydrate, aluminum zirconium tetrachlororhydrate, aluminum zirconium pentachlorohydrate, aluminum zirconium octachlorohydrate, aluminum zirconium trichlorohydrex glycine complex, aluminum zirconium tetrachlorohydrex glycine complex, aluminum zirconium octachlororhydrex glycine complex, aluminum chloride, aluminum sulfate buffered, and combinations thereof.

Commercially available products include for example: Aluminum-zirconium chloride-propylene glycol complexes include "REZAL 36 GPG Powder", "REACH AZP-908 PG Powder" (each, product of Reheis, Inc.), and "Westchlor ZR41 PG ASP Powder (product of Westwood Chemical), while aluminum-zirconium chloride-glycine complexes include "REACH AZP-908", "REACH AZP-908SUF", and "REACH AZP-902" (each, product of Reheis, Inc.).

### Structurant

The topical stick composition of the present invention contains a structurant selected from steryl alcohol, cetyl alcohol, arachidyl alcohol, behenyl alcohol and hydrogenated castor oil, and mixtures thereof.

The structurant material contributes to the stick form with the desired viscosity, rheology, texture and/or hardness, or to otherwise help suspend any dispersed solids or liquids within the composition. The structurant is preferably present in the compositions of the present invention from 3% to 35%, more preferably from 3% to 30%, even more preferably from 3% to 25%, by weight of the total composition.

The term "structurant" as used herein is effective in providing solidifying, gelling, viscosifying, thickening and/or suspending properties to the composition or which otherwise provides structure to the final product form. These structurants will typically be solids under ambient conditions. It is preferred that structurants have a melting point of at least about 40°C and are solids when at or below body temperature.

Commercial examples of structurants include CO-1985 (The Procter & Gamble Company), NACOL 20-95, NACOL 22-98 and NAFOL 1822B (Sasol Corp., Germany), and a preferred hydrogenated castor oil include Castorwax MP80 (Caschem Inc.), as well as many others. Additional examples can be found in the CTFA International Cosmetic Ingredient Dictionary.

### Other ingredients

### Liquid carrier

The topical stick compositions of the present invention optionally contain a liquid carrier at concentrations ranging preferably from 10% to 99%, more preferably from 20% to 70%, by weight of the composition. Such concentrations will vary depending upon variables such as desired product hardness, selection of other ingredients in the composition, and so forth. The liquid carrier for use in the composition can be any aqueous or anhydrous liquid that is known for use in personal care applications or is otherwise suitable for topical application to the skin.

The liquid carrier preferably includes a volatile silicone liquid, which may include cyclic, linear and/or branched chain silicones. The concentration of volatile silicone in the topical stick composition of the present invention preferably ranges from 5% to 80%, preferably from 10% to 60%, more preferably from 15% to 60%, by weight of the composition. The volatile silicone is preferably a cyclic silicone having from about 3 to 7, more preferably from about 4 to 6, even more preferably 5, silicon atoms. Most preferred are those that conform to the formula: 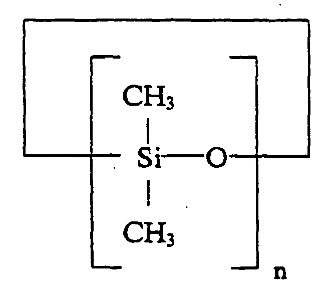 wherein n is from about 3 to 7, preferably from about 4 to 6, even more preferably 5. These volatile cyclic silicones generally have a viscosity of less than about 10 centistokes. Suitable volatile silicones for use herein include, but are not limited to, Dow Coming 244, Dow Coming 245, Dow Coming 344, and Dow Coming 345 (commercially available from Dow Coming Corp.); and Silicone Fluids SF-1202 and SF-1173 (available from General Electric Co.); and KF994 and KF995 (available from Shin Etsu Co.). Non limiting examples of suitable volatile silicones are described in Todd et al., "Volatile Silicone Fluids for Cosmetics", Cosmetics and Toiletries, 91:27-32 (1976).

The liquid carrier may comprise a non-volatile silicone liquid, preferred concentrations of which range from 1% to 35%, more preferably from 2% to 20%, by weight of the composition. The non-volatile silicone carrier is preferably a liquid at or below human skin temperature (e.g. about 37°C), or otherwise in liquid form within the composition during or shortly after topical application.

The non-volatile silicone fluid may be a polyalkyl siloxane, a polyalkylaryl siloxane or a polyether siloxane copolymer. The essentially non-volatile polyalkyl siloxanes that may be used include, for example, polydimethyl siloxanes with viscosities ranging from about 5 to 100,000 centistokes at 25°C. Also preferred are those non-volatile polydimethyl siloxanes that conform to the formula: 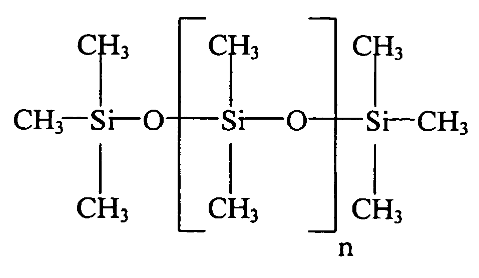 wherein n is sufficiently large to provide a viscosity of up to about 100,000 centistokes, preferably less than about 500 centistokes, more preferably from about 5 centistokes to 200 centistokes, even more preferably from about 5 centistokes to 50 centistokes, as measured under ambient conditions. Specific non-limiting examples of suitable non-volatile, linear, silicone carriers include Dow Coming 200, Dow Coming 225 (available from Dow Coming Corp.); SF-96 and SF-18(350) (available from G.E. Silicones); and DM-Fluid (available from Shin Etsu Co.).

These polyalkylaryl siloxanes are available, for example, as SF-1550 (available from G.E. Silicones), 556 Cosmetic Grade Fluid (available from Dow Coming Corp.) or KF56 (available from Shin Etsu Co.). The essentially non-volatile polyether siloxane copolymer that may be used is for example, a dimethyl polyoxyalkylene ether copolymer fluid having a nominal viscosity of about 1200 to 1500 centistokes at 25°C. This copolymer is available, for example, as SF-1188, SF-1288 (available from G.E. Silicones), KF6017 (available from Shin Etsu Co.). Preferred compounds of this type are polysiloxane ethylene glycol ether copolymers.

Many other liquid carriers known for use in personal care products can be used in the topical compositions, alone or in combination with other liquid carriers including those described in more detail herein. Many such other liquid carriers are disclosed in U.S. Pat. Nos. 6,013,248 (Luebbe et al.) and 5,968,489 (Swaile et al.).

Examples of other liquid carriers include PPG-14 butyl ether (a condensate of about 14 moles of propylene oxide with about 1 mole of butyl alcohol, e.g. Fluid AP, available from Union Carbide and PROBTYL 14, available from Croda), isopropyl myristate, isopropyl palmitate, cetyl octanoate, C₁₂-C₁₅ alkylbenzoate (e.g., Finsolv, available from Fintex Inc.), octyldodecanol, isostearyl isostearate, octadodecyl benzoate, isostearyl lactate, isostearyl palmitate, isobutyl stearate, mineral oil, and volatile isoparaffin, and mixtures thereof.

Preferred liquid carriers include one or more selected from the group consisting of volatile silicones, non-volatile silicones, PPG-14 butyl ether, C₁₂₋₁₅ alkyl benzoate, and mixtures thereof.

### Emollient

The topical stick composition of the present invention optionally contains emollients. Non-limiting examples of emollients preferably include a semi-solid or paste under ambient conditions, which can be formulated as a liquid, semi-solid, or solid within the topical stick composition.

Preferred emollients are for example petrolatum, butyl stearate, butyl myristate and myristyl myristate, cholesterol and its derivatives such as cholesteryl isostearate and ceramide and its analogues. More preferred is petrolatum. The petrolatum can be derived from fractional distillation of still residues from the steam distillation of paraffin-based petroleum, or from steam- reduced crude oils from which the light fractions have been removed. The petrolatum for use in the compositions of the present invention is preferably U.S.P. white petrolatum. Especially preferred is petrolatum containing relatively low levels of alkyl chain lengths having less than 26 carbon atoms, such as Witco Super White Protopet.

The liquid carriers described above may also function as emollients in the topical stick compositions of the present invention.

### Additional Other Ingredients

The topical compositions of the present invention may further contain any optional ingredient that is known for use in antiperspirants and deodorant products or other personal care products, or which is otherwise suitable for topical application to human skin.

Non limiting examples of optional ingredients include dyes or colorants, emulsifiers, perfumes, deodorant perfumes, antimicrobial or other deodorant materials, preservatives, vitamins, anti-oxidants, coupling agents or other solvents, surfactants, processing aides such as viscosity modifiers, wash-off aids, distributing agents, pharmaceutical agents, fillers, botanical extracts, and so forth. Examples of such optional materials are described in U.S. Pat. No. 4,049,792 (Elsnau); U.S. Pat. No. 5,019,375 (Tanner et al.); and U.S. Pat. No. 5,429,816 (Hofrichter et al.).

The composition may contain 0.0001-8%, preferably 0.001-6%, more preferably 0.005-5% by weight of the total composition of preservatives. A variety of preservatives are suitable, including benzoic acid, benzyl alcohol, benzylhemiformal, benzylparaben, 5-bromo-5- nitro-1,3-dioxane, 2-bromo-2-nitropropane-1,3-diol, butyl paraben, calcium benzoate, calcium propionate, captan, chlorhexidine diacetate, chlorhexidine digluconate, chlorhexidine dihydrochloride, chloroacetamide, chlorobutanol, p-chloro-m-cresol, chlorophene, chlorothymol, chloroxylenol, m-cresol, o-cresol, DEDM Hydantoin, DEDM Hydantoin dilaurate, dehydroacetic acid, diazolidinyl urea, dibromopropamidine diisethionate, DMDM Hydantoin, and all of those disclosed on pages 570 to 571 of the CTFA Cosmetic Ingredient Handbook, Second Edition, 1992.

The compositions of the invention may contain vitamins and/or coenzymes, as well as antioxidants. If so, 0.001-10%, preferably 0.01-8%, more preferably 0.05-5% by weight of the total composition are suggested. Suitable vitamins include the B vitamins such as thiamine, riboflavin, pyridoxin, and so on, as well as coenzymes such as thiamine pyrophoshate, flavin adenin dinucleotide, folic acid, pyridoxal phosphate, tetrahydrofolic acid, and so on. Also Vitamin A and derivatives thereof are suitable. Examples include Vitamin A palmitate, acetate, or other esters thereof, as well as Vitamin A in the form of beta carotene. Also suitable is Vitamin E and derivatives thereof such as Vitamin E acetate, nicotinate, or other esters thereof. In addition, Vitamins D and K are suitable.

Suitable antioxidants are ingredients which assist in preventing or retarding spoilage. Examples of antioxidants suitable for use in the compositions of the invention include potassium sulfite, sodium bisulfite, sodium erythrobate, sodium metabisulfite, sodium sulfite, propyl gallate, cysteine hydrochloride, butylated hydroxytoluene, butylated hydroxyanisole, and so on.

Fillers and particulate materials such as for example talc, mica, sericite, kaolin, starch, hydrophobic starch such as DRY FLO PC (National Starch), Silicone resin such as Tospearl (GE Toshiba Silicone, Japan), fumed or colloidal silica such as CAB-O-Sil M-5 (Cabot Corp.), and nonfumed silica as well as mixtures thereof may also be included. This filler material enhances the aesthetic characteristics of the solid sticks and may serve to stabilize the structure of such sticks. The topical compositions may contain from 0.01% to 10% of filler material, by weight of the composition.

The compositions of the present invention may also include a clay component as a filler, as well as an activator for the clay. The clays are preferably generally present from 0.5% to 7%, more preferably from 1% to 5%, by weight of the total composition.

Clay materials suitable for use in the compositions of the present invention are preferably selected from the group consisting of montmorillonite clays and hydrophobically treated montmorillonite clays, and mixtures thereof. Montmorillonite clays are those which contain the mineral montmorillonite and are characterized by having a suspending lattice. Examples of these clays include the bentonites, hectorites, and colloidal magnesium aluminum silicates.

Bentonite is a colloidal, hydrated aluminum silicate obtained from montmorillonite and has the formula Al₂O₃·4SiO₂·H₂O. It is commercially available under the tradename of "Bentone." Specific examples of Bentones are Bentone 38, Bentone 34, Bentone 27, Bentone 14, and Bentone LT, all of which have a particle size of below about 5 microns and are commercially available from Elementis Specialties. Bentone 38 is particularly preferred. A more detailed discussion of bentonites can be found in the Kirk-Othmer Encyclopedia of Chemical Technology, 2nd Ed., Vol. 3 (1964), pp. 339-360, published by Interscience Publishers.

Hectorite, also a montmorillonite clay, differs from bentonite in that there is almost a complete substitution of aluminum in the lattice structure of bentonite by magnesium. In addition, hectorites contain lithium and fluorine. Another preferred clay material for use in the present invention is quatemium-18 hectorite.

The magnesium aluminum silicates are complexes of colloidal magnesium aluminum silicate richer in magnesium than aluminum. Magnesium aluminum silicates are commercially available as Veegum (R.T. Vanderbilt Co.).

When used, the activators for the clay are preferably present in the composition from 0.2% to 1.5%, more preferably from 0.25% to 1.25%, by weight of the total composition. The activators are generally polar compounds which chemically activate the clay materials. Examples of such polar activators include propylene carbonate, ethanol, and mixtures of those materials. Ethanol is preferred since it enhances the composition's ability to incorporate water. The clays and activators may be incorporated separately or may be purchased as a mixture of clay, activator and a solvent. For example, Bentone Gel VSSV, commercially available from Elementis Specialties, is a mixture of about 77% cyclomethicone, 18% quaternium-18 hectorite and 5% SDA-40 alcohol (ethanol).

The compositions of the present invention may optionally contain porous inorganic particles as a filler. A preferred porous inorganic particle includes calcium silicate and silica. The preferred pore volume is at least 2ml/g. Porous particles may contain perfume absorbed into the particle. Porous particles containing perfume which may be used include those described in U.S. Pat. No. 5,336,665 (Garner-Gray et al.). Examples of such porous particles include FLORITE R (Tokuyama Corp., Japan) and SUNSPHERE H-33 (Asahi Glass Corp., Japan). The topical stick composition preferably contains from 0.02% to 10%, more preferably from 0.05% to 5% by weight of a porous particle.

Examples of botanical extracts which may be included in the compositions of the present invention, include ginger root, ginger rhizome, almond, birch, clove, rose hip, white birch, gambi, burnet, hiba, willow herb, Phellodendron Amurense, Coptis Chinesis, clove oil extract, tea tree oil, olive leaf extract, rosemary extract, fennel seed, Scutellaria Root, Horse Chestnut, and Ginkgo Biloba. Preferred is Phellodendron Amurense.

The compositions of the present invention may also include pharmaceutical agents. Examples of pharmaceutical agents include phytoplenolin, sericin, aesculin, baicalin, wogonin, pantothenic acid and its derivatives such as panthenyl triacetate, K2 glycerrizinate, capsaicin, menthol, menthyl lactate, hydrocortisone, allantoin, stearyl glycyrrhetinate, and guaiazulene

The compositions of the present invention may also include deodorant embodiments which preferably contain a deodorant active, perfume or combinations of those materials, at concentrations ranging from 0.01% to 60%, more preferably from 0.01 % to 20%, even more preferably from 0. 01% to 10%, and even more preferably from 0.1% to 0.5%, by weight of the composition. These deodorant actives and perfumes include any known or otherwise safe and effective deodorant active or perfume suitable for topical application to human skin.

Deodorant actives suitable for use in the embodiments of the present invention include any topical material that is known for or is otherwise effective in preventing or eliminating malodor associated with perspiration. These deodorant actives are typically antimicrobial agents (e.g., bactericides or fungicides), malodor-absorbing materials, or combinations thereof.

Preferred deodorant actives are antimicrobial agents, non-limiting examples of which include acetyltrimethylammonium bromide, cetyl pyridinium chloride, benzethonium chloride, diisobutyl phenoxy ethoxy ethyl dimethyl benzyl ammonium chloride, sodium N-lauroyl sarcosine, sodium N-palmitoyl sarcosine, N-myristoyl glycine, potassium N-lauroyl sarcosine, trimethyl ammonium chloride, sodium aluminum chlorohydroxy lactate, triethyl citrate, tricetylmethyl ammonium chloride, 2,4,4'-trichloro-2'-hydroxy diphenyl ether (triclosan), 3,4,4'- trichlorocarbanilide (triclocarban), diaminoalkyl amides such as L-lysine hexadecyl amide, heavy metal salts of citrate, salicylate, and piroctose, especially zinc salts, and acids thereof, heavy metal salts of pyrithione, especially zinc pyrithione, zinc phenolsulfate, farnesol, phenoxyethanol, and combinations thereof.

Preferred deodorant actives are triclosan, triclocarban and combinations thereof, wherein the preferred concentration of either triclosan or triclocarban ranges from 0.01% to 10%, more preferably from 0.1% to 5%, even more preferably from 0. 1% to 3%, by weight of the composition, and wherein the total concentration of triclosan and triclocarban when used together in a composition ranges from 0.01% to 2%, more preferably from 0.2% to 1%, even more preferably from 0.2% to 0.6%, by weight of the composition.

Other deodorant actives include odor-absorbing materials such as carbonate and bicarbonate salts, including alkali metal carbonates and bicarbonates, ammonium and tetraalkylammonium. Preferred are sodium and potassium salts of such odor-absorbing materials. Still other deodorant actives include the antiperspirant actives described hereinbefore.

Perfumes suitable for use in the embodiments of the present invention include any perfume material that can be applied to the skin and is known for or is otherwise effective in masking malodor associated with perspiration, or which otherwise provides the composition with the desired perfumed aroma. These include any perfume or perfume chemical, including pro-perfumes and deo-perfumes, suitable for topical application to the skin. The amount or concentration of the perfume in the deodorant embodiments should be effective to provide the desired aroma characteristics or to mask malodor, wherein the malodor is inherently associated with the composition itself or is associated with malodor development from human perspiration.

Perfumes are made by those skilled in the art in a wide variety of fragrances and strengths. Typical perfumes and fragrances are described in Arctander, Perfume and Flavour Chemicals (Aroma Chemicals), Vol. I and II (1969); and Arctander, Perfume and Flavour Materials of Natural Origin (1960); U.S. Pat. No. 4,322,308 (Hooper et al.); U.S. Pat. No. 4,304,679 (Hooper et al.); U.S. Pat. No. 5,554,588 (Behan at al.); U.S. Pat. No. 4,278,658 (Hooper et al.); U.S. Pat. No. 5,501,805 (Behan et al.); and EP Patent Application 684 037 A1.

The topical stick composition also may comprise various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, purple, etc. Organic pigments generally consist of insoluble metallic salts of certified color additives, referred to as the Lakes. Inorganic pigments include iron oxides, ultramarines, chromium, chromium hydroxide colors, and mixtures thereof.

### Method of Manufacture

The topical compositions of the present invention may be prepared by any known or otherwise effective technique suitable for formulating the desired product form. For example, volatile and/or nonvolatile liquid carriers, unsaturated fatty acid and structurants are mixed and heated sufficiently to liquefy the structurants, e.g., approximately 80°C for many wax solids, to form a single phase liquid. Antiperspirant active, and optionally fillers, are then typically added to and dispersed throughout the heated, single-phase liquid before allowing the resulting combination to cool to approximately 70°C, at which point perfumes and similar other materials (if any) are mixed into the combination which is then cooled to just above the solidification point of the structurants (e.g., typically about 65°C) and then poured into dispensing packages and allowed to solidify under ambient conditions or cooled conditions.

### Method of Use

The topical stick compositions of the present invention may be applied topically to the axilla or other area of the skin in an amount effective to treat or reduce perspiration wetness and/or malodor. The composition is preferably applied in an amount ranging from about 0.1-10 grams, more preferably from about 0.1 to 5 grams, and even more preferably from about 0.1 to 1 gram, to the desired area of the skin. The compositions are preferably applied one or two times daily, preferably once daily, to achieve effective control of antiperspirant and/or malodor.

### EXAMPLES

The following examples further describe and demonstrate embodiments of the present invention. The examples are given solely for the purpose of illustration.

### Example 1

Four types of topical stick formulations were prepared. The compositions are shown in Table 1. The liquid carriers such as PPG-14 butyl ether and C₁₂₋₁₅ alkyl benzoate, the unsaturated fatty acid such as Oleic acid, and the structurant components such as Stearyl alcohol, Hydrogenated Castor Oil, and Behenyl alcohol, are weighed out and placed in a 2000 ml beaker. The mixture of ingredients in the 2000 ml beaker is heated while stirring, which melts the mixture evenly. Heating and stirring are continued until the temperature is about 80°C. The mixture is cooled to about 75°C and cyclomethicone is the added while stirring. Then, the filler such as talc and the antiperspirant active salt are mixed in while maintaining the temperature at 75°C. After the resulting mixture was stirred for 60 minutes to break-up and mechanically disperse any clumps of powder materials, the mixture is further cooled to about 63°C. Fragrance is added and mixing is continued for another 5 minutes. The mixture is poured into plastic containers of the type normally used for antiperspirant/deodorant products and placed in a refrigerator at about 5°C for about 20 minutes. Cooling is completed at room temperature.

| **Table 1** | Comparison #1 | Example #1 | Example #2 | Comparison #2 |
|---|---|---|---|---|
| Ingredient | % | % | % | % |
| Stearyl Alcohol | 16.00 | 16.00 | 16.00 | 16.00 |
| Behenyl Alcohol | 0.60 | 0.60 | 0.60 | 0.60 |
| Hydrogenated Castor Oil | 3.25 | 3.25 | 3.25 | 3.25 |
| PPG-14 Butyl Ether | 3.00 | 3.00 | 3.00 | 3.00 |
| C12-15 Alkyl Benzoate | 4.00 | 4.00 | 4.00 | 4.00 |
| Cyclopentasiloxane | balance | balance | balance | balance |
| Aluminum Zirconium Tetrachlorohydrex Gly | 24.00 | 24.00 | 24.00 | 24.00 |
| Talc | 7.00 | 7.00 | 7.00 | 7.00 |
| Oleic acid | | 0.20 | 0.50 | 1.00 |
| Fragrance | 0.50 | 0.50 | 0.50 | 0.50 |
| Total | 100.0 | 100.0 | 100.0 | 100.0 |
| Stick hardness (grams) | | | | |
| Measurement #1 | 512.6 | 506.6 | 543.8 | 473.8 |
| Measurement #2 | 445.6 | 568.6 | 551.2 | 466.2 |
| Measurement #3 | 496.2 | 585.4 | 594.0 | 478.8 |
| Measurement #4 | 516.6 | 601.8 | 514.4 | 500.8 |
| Measurement #5 | 399.0 | 556.6 | 507.0 | 433.4 |
| Measurement #6 | 512.8 | 517.0 | 464.4 | 438.4 |
| Average of 6 measurements | 480.5 | 556.0 | 529.1 | 465.2 |
| Standard deviation | 47.9 | 37.6 | 44.3 | 25.5 |
| Relative hardness | 100.0% | 115.7% | 110.1 % | 96.8% |
| Aesthetics of stick | | | | |
| Use feel on application | Control | Much drier than control | Drier than control | Almost same as control |
| Whiteness after dry down | Control | Less white than control | Less white than control | Less white than control |
| Smell score on application | 1 | 1 | 2 | 3 |

In Comparison #1, the formulation does not contain any oleic acid. In Comparison #2, the formulation contains 1% of oleic acid. In Examples #1 and #2, which are exemplary as embodiments of the present invention, the formulations contain 0.2% and 0.5% of oleic acid, respectively.

The hardness of the four types of sticks was measured. The "hardness" as measured herein represents the peak force required to move a penetration plunger into a topical stick composition for a specified distance and at a controlled rate under the following test conditions, wherein higher values represent a harder product, and lower values represent a softer product.

In a typical method known in the art, these hardness values were measured at 25°C, 40% relative humidity, using a FUDOH RHEO METER NRM-2010J-CW, available from RHEOTECH Corp., Bunkyo-Ku, Tokyo, JAPAN. The standard penetration plunger used was smooth, made of stainless steel and had a diameter of 3 mm. It is available from RHEOTECH Corp., as adapter number 6. The hardness value represents the peak force required to move the standard plunger from the surface through the core of the stick composition, for a distance of 10 mm at a rate of 2 cm/minute. Six stick samples of each type of topical stick compositions were prepared for the measurement of hardness. Each stick was submitted to one measurement. The hardness value of the stick composition was calculated as an average of the six values.

The relative hardness is calculated as a ratio of the hardness of the stick composition to the hardness of a "base stick". The hardness of the base stick is assumed to be 100 %. In Table 1, the base stick is "Comparison #1".

As shown in Table 1, the relative hardness of Example #1 is 115.7% and Example #2 is 110.1%, which provides a surprising improvement in hardness value versus the base stick. Furthermore, in Comparison #2 wherein the stick contains 1% of oleic acid, the relative hardness is 96.8%, which indicates that Comparison #2 is softer than the base stick. Examples #1 and #2 are also stable, provide good antiperspirant performance with good skin feel and minimized whiteness after dry down.

Using the above evaluation, it is preferred that the relative hardness of the topical stick compositions of the present invention has a value of at least about 103%, more preferably at least about 105%, even more preferably at least about 107%, and even more preferably at least about 110%.

In addition, an odor evaluation was performed to evaluate the smell score on application to determine if there were any unpleasant odors coming from the product. An expert odor evaluator first applied a typical amount of a stick composition onto his/her forearm. Then the evaluator sniffed the applied area immediately and evaluated the odor according to the following scale:

| | |
|---|---|
| 0: | No smell |
| 1: | Hardly smells |
| 2: | Faintly smells |
| 3. | Slightly smells |
| 4. | Smells |
| 5. | Clearly smells |
| 6. | Strongly smells |

On this scale, the score of 0, 1, 2 and 3 are "acceptable", while 4, 5 and 6 are "not acceptable". As shown also in Table 1, even with the addition of oleic acid at 0.2% and 0.5%, the smell score on application is still in the acceptable range.

### Example 2

The following Examples 2-A, 2-B, and 2-C illustrate example topical stick composition embodiments. The examples are prepared by methods well known for making topical stick compositions. All percentages are by weight.

### Example 2-A

| | (1) | (2) |
|---|---|---|
| Ingredients | % | % |
| Stearyl Alcohol | 16 | 16 |
| Arachidyl Alcohol | | 2 |
| Behenyl Alcohol | 0.6 | |
| Hydrogenated Castor Oil | 3.25 | 3.5 |
| PPG14-BE | 3 | 4 |
| C12-15 Alkyl Benzoate | 4 | 7 |
| Oleic acid | 0.4 | 0.7 |
| Cyclomethicone | 40.95 | 35.1 |
| Aluminum Zirconium Tetrachlorohydrex GLY | 24 | 24 |
| Talc | 7 | 7 |
| Calcium Silicate (FLORITE R®) | 0.3 | |
| Porous Silica (SUNSPHERE H-33®) | | 0.2 |
| Fragrance | 0.5 | 0.5 |
| Total | 100 | 100 |

### Example 2-B

| | (1) | (2)* |
|---|---|---|
| Ingredients | % | % |
| Stearyl Alcohol | 16 | 16 |
| Behenyl Alcohol | 0.6 | 0.6 |
| Ceresin | 3 | 3 |
| Petrolatum | 1 | 1 |
| PPG14-BE | 1 | 1 |
| Dimethicone, 10cs | 5 | 5 |
| Oleic acid | 0.5 | |
| Linoleic acid | | 0.15 |
| Cyclomethicone | 48.15 | 48.5 |
| Aluminum Zirconium Tetrachlorohydrex GLY | 24 | 24 |
| Porous Silica (SUNSPHERE H-33®) | 0.25 | 0.25 |
| Fragrance | 0.5 | 0.5 |
| Total | 100 | 100 |

| | | |
|---|---|---|
| *: Example 2-B(2) is outside the scope of present invention and serves as a reference. | | |

### Example 2-C

| **(1)** | |
|---|---|
| Ingredients | % |
| Stearyl Alcohol | 15 |
| Behenyl Alcohol | 0.2 |
| Hydrogenated Castor Oil | 4.5 |
| Phenyl Trimethicone | 10 |
| Mineral oil | 5 |
| Oleic acid | 0.3 |
| Cyclomethicone | 40.3 |
| Aluminum Zirconium Tetrachlorohydrex GLY | 24 |
| Fumed Silica (CAB-O-SIL M-5®) | 0.2 |
| Fragrance | 0.5 |
| Total | 100 |

## Claims

1. A topical stick composition comprising by weight of the composition:
from 0.5% to 60% of an antiperspirant active;
from 3% to 35% of a structurant selected from stearyl alcohol, cetyl alcohol, arachidyl alcohol, behenyl alcohol and hydrogenated castor oil, and mixtures thereof; and
from 0.2% to 0.7% of an unsaturated fatty acid.

2. The topical stick composition of Claim 1, wherein the unsaturated fatty acid is a carboxylic acid having from about 9 to about 30 carbon atoms.

3. The topical stick composition of Claim 2, wherein the carboxylic acid is selected from the group consisting of oleic acid, linoleic acid, linolenic acid, palmitoleic acid, and mixtures thereof.

4. The topical stick composition of Claim 3, wherein the carboxylic acid is selected from the group consisting of oleic acid, linoleic acid, linolenic acid, and mixtures thereof.

5. The topical stick composition of Claim 1, wherein the composition is anhydrous.

6. The topical stick composition of Claim 1, wherein the composition further comprises from 10% to 80% by weight of a liquid carrier.

7. The topical stick composition of Claim 6, wherein the carrier is a volatile or nonvolatile solvent.

8. The topical stick composition of Claim 7, wherein the solvent is a silicone.

9. The topical stick composition of Claim 1, wherein the composition further comprises a filler.

10. The topical stick composition of Claim 1, wherein the composition further comprises a botanical extract.

11. The topical stick composition of Claim 1, wherein the antiperspirant active is selected from the group consisting of organic or inorganic salts of aluminum, zirconium, zinc, and mixtures thereof.

12. The topical stick composition of claim 1 comprising by weight of the composition:
from 5% to 40% of an antiperspirant active selected from the group consisting of aluminum, zirconium, zinc salts, and mixtures thereof;
from 3% to 30% of a structurant selected from the group consisting of stearyl alcohol, cetyl alcohol, arachidyl alcohol, behenyl alcohol, hydrogenated castor oil, and mixtures thereof; and
from 0.2% to 0.7% of an unsaturated fatty acid selected from the group consisting of oleic acid, linoleic acid, linolenic acid, and mixtures thereof.

13. The topical stick composition of Claim 12, wherein the composition is anhydrous.

14. The topical stick composition of Claim 12, comprising from 0.2% to 0.6% by weight of the unsaturated fatty acid.

15. The topical stick composition of Claim 12, wherein the unsaturated fatty acid is oleic acid.

16. The topical stick composition of Claim 12, further comprising from 10% to 60% of a liquid carrier.

17. The topical stick composition of Claim 16, wherein the liquid carrier is selected from the group consisting of volatile silicones, non-volatile silicones, PPG-14 butyl ether, C₁₂₋₁₅ alkyl benzoate, and mixtures thereof.

18. The topical stick composition of Claim 12, further comprising a calcium silicate containing a perfume absorbed within.

## Patentansprüche

1. Topische Stiftzusammensetzung, umfassend, bezogen auf das Gewicht der Zusammensetzung:
0,5 bis 60 % eines schweisshemmenden Mittels,
3 bis 35 % eines strukturgebenden Mittels, ausgewählt aus Stearylalkohol, Cetylalkohol, Arachidylalkohol, Behenylalkohol und hydriertem Rizinusöl und Mischungen davon, und
0,2 bis 0,7 % einer ungesättigten Fettsäure.

2. Topische Stiftzusammensetzung gemäss Anspruch 1, wobei die ungesättigte Fettsäure eine Carbonsäure mit etwa 9 bis etwa 30 Kohlenstoffatomen ist.

3. Topische Stiftzusammensetzung gemäss Anspruch 2, wobei die Carbonsäure ausgewählt ist aus der Gruppe bestehend aus Ölsäure, Linolsäure, Linolensäure, Palmitoleinsäure und Mischungen davon.

4. Topische Stiftzusammensetzung gemäss Anspruch 3, wobei die Carbonsäure ausgewählt ist aus der Gruppe bestehend aus Ölsäure, Linolsäure, Linolensäure und Mischungen davon.

5. Topische Stiftzusammensetzung gemäss Anspruch 1, wobei die Zusammensetzung wasserfrei ist.

6. Topische Stiftzusammensetzung gemäss Anspruch 1, wobei die Zusammensetzung ferner 10 bis 80 Gew.% eines flüssigen Trägers umfasst.

7. Topische Stiftzusammensetzung gemäss Anspruch 6, wobei der Träger ein flüchtiges oder nicht-flüchtiges Lösungsmittel ist.

8. Topische Stiftzusammensetzung gemäss Anspruch 7, wobei das Lösungsmittel ein Silicon ist.

9. Topische Stiftzusammensetzung gemäss Anspruch 1, wobei die Zusammensetzung ferner einen Füllstoff umfasst.

10. Topische Stiftzusammensetzung gemäss Anspruch 1, wobei die Zusammensetzung ferner einen Pflanzenextrakt umfasst.

11. Topische Stiftzusammensetzung gemäss Anspruch 1, wobei das schweisshemmende Mittel ausgewählt ist aus der Gruppe bestehend aus organischen Salzen von Aluminium, Zirkonium, Zink und Mischungen davon.

12. Topische Stiftzusammensetzung gemäss Anspruch 1, umfassend, bezogen auf das Gewicht der Zusammensetzung:
5 bis 40 % eines schweisshemmenden Mittels, ausgewählt aus der Gruppe bestehend aus Aluminium-, Zirkonium-, Zinksalzen und Mischungen davon,
3 bis 30 % eines strukturgebenden Mittels, ausgewählt aus der Gruppe bestehend aus Stearylalkohol, Cetylalkohol, Arachidylalkohol, Behenylalkohol, hydriertem Rizinusöl und Mischungen davon, und
0,2 bis 0,7 % einer ungesättigten Fettsäure, ausgewählt aus der Gruppe bestehend aus Ölsäure, Linolsäure, Linolensäure und Mischungen davon.

13. Topische Stiftzusammensetzung gemäss Anspruch 12, wobei die Zusammensetzung wasserfrei ist.

14. Topische Stiftzusammensetzung gemäss Anspruch 12, umfassend 0,2 bis 0,6 Gew.% der ungesättigten Fettsäure.

15. Topische Stiftzusammensetzung gemäss Anspruch 12, wobei die ungesättigte Fettsäure Ölsäure ist.

16. Topische Stiftzusammensetzung gemäss Anspruch 12, ferner umfassend 10 bis 60 % eines flüssigen Trägers.

17. Topische Stiftzusammensetzung gemäss Anspruch 16, wobei der flüssige Träger ausgewählt ist aus der Gruppe bestehend aus flüchtigen Siliconen, nichtflüchtigen Siliconen, PPG-14-Butylether, C₁₂₋₁₅-Alkylbenzoat und Mischungen davon.

18. Topische Stiftzusammensetzung gemäss Anspruch 12, ferner umfassend ein Calciumsilicat, das darin absorbiert ein Parfüm enthält.

## Revendications

1. Composition topique en bâton comprenant en poids de la composition :
de 0,5 % à 60 % d'un actif antisudorifique ;
de 3 % à 35 % d'un structurant
choisi parmi l'alcool stéarylique, l'alcool cétylique, l'alcool d'arachidylique, l'alcool béhénylique et l'huile de ricin hydrogénée, et les mélanges de ceux-ci ; et
de 0,2 % à 0,7 % d'un acide gras insaturé.

2. Composition topique en bâton selon la revendication 1, dans laquelle l'acide gras insaturé est un acide carboxylique ayant d'environ 9 à environ 30 atomes de carbone.

3. Composition topique en bâton selon la revendication 2, dans laquelle l'acide carboxylique est choisi dans le groupe constitué de l'acide oléique, l'acide linoléique, l'acide linolénique, l'acide palmitoléique, et les mélanges de ceux-ci.

4. Composition topique en bâton selon la revendication 3, dans laquelle l'acide carboxylique est choisi dans le groupe constitué de l'acide oléique, l'acide linoléique, l'acide linolénique, et les mélanges de ceux-ci.

5. Composition topique en bâton selon la revendication 1, dans laquelle la composition est anhydre.

6. Composition topique en bâton selon la revendication 1, dans laquelle la composition comprend en outre de 10 % à 80 % en poids d'un support liquide.

7. Composition topique en bâton selon la revendication 6, dans laquelle le support est un solvant volatil ou non volatil.

8. Composition topique en bâton selon la revendication 7, dans laquelle le solvant est un silicone.

9. Composition topique en bâton selon la revendication 1, dans laquelle la composition comprend en outre une charge.

10. Composition topique en bâton selon la revendication 1, dans laquelle la composition comprend en outre un extrait végétal.

11. Composition topique en bâton selon la revendication 1, dans laquelle l'actif antisudorifique est choisi dans le groupe constitué des sels organiques ou inorganiques d'aluminium, de zirconium, de zinc, et des mélanges de ceux-ci.

12. Composition topique en bâton selon la revendication 1, comprenant en poids de la composition :
de 5 % à 40 % d'un actif antisudorifique choisi dans le groupe constitué des sels d'aluminium, de zirconium, de zinc, et des mélanges de ceux-ci ;
de 3 % à 30 % d'un structurant choisi dans le groupe constitué de l'alcool stéarylique, l'alcool cétylique, l'alcool d'arachidylique, l'alcool béhénylique et l'huile de ricin hydrogénée, et des mélanges de ceux-ci ; et
de 0,2 % à 0,7 % d'un acide gras insaturé choisi dans le groupe constitué de l'acide oléique, l'acide linoléique, l'acide linolénique, et des mélanges de ceux-ci.

13. Composition topique en bâton selon la revendication 12, dans laquelle la composition est anhydre.

14. Composition topique en bâton selon la revendication 12, comprenant de 0,2 % à 0,6 % en poids de l'acide gras insaturé.

15. Composition topique en bâton selon la revendication 12, dans laquelle l'acide gras insaturé est l'acide oléique.

16. Composition topique en bâton selon la revendication 12, comprenant en outre de 10 % à 60 % d'un support liquide.

17. Composition topique en bâton selon la revendication 16, dans laquelle le support liquide est choisi dans le groupe constitué de silicones volatiles, silicones non volatiles, éther butylique de PPG-14, benzoate d'alkyl en C₁₂₋₁₅, et des mélanges de ceux-ci.

18. Composition topique en bâton selon la revendication 12, comprenant en outre un silicate de calcium contenant un parfum absorbé à l'intérieur.
